# EUROPÄISCHE PATENTSCHRIFT

(11) **EP 3 569 749 B1**
(45) Veröffentlichungstag und Bekanntmachung des Hinweises auf die Patenterteilung: **06.09.2023**
(21) Anmeldenummer: 19164052.3
(22) Anmeldetag: 20.03.2019
(51) Int. Cl.: D04B 1/26, D04B 1/28

(54) **NAHTLOSER KOMPRESSIONSARTIKEL**
SEAMLESS COMPRESSION ARTICLE
ARTICLE DE COMPRESSION SANS COUTURE

(30) Priorität: 17.05.2018 DE 202018102766 U
(43) Veröffentlichungstag der Anmeldung: 20.11.2019
(60) Teilanmeldung: 22195608.9
(73) Patentinhaber: Julius Zorn GmbH, 86551 Aichach (DE)
(72) Erfinder: Lechner, Siegfried, 86529 Schrobenhausen (DE)
(74) Vertreter: Charrier Rapp & Liebau

(56) Entgegenhaltungen:
- WO-A1-2012/067645
- WO-A1-2013/123038
- DE-C2- 10 084 580
- US-A- 1 941 508
- US-A- 3 905 212

## Beschreibung

Die Erfindung betrifft einen auf einer Flachstrickmaschine gestrickten nahtlosen Kompressionsartikel nach dem Oberbegriff des Anspruchs 1.

Nahtlos gestrickte Kompressionsartikel sind aus dem Stand der Technik bekannt. Aus der US 3905 212 B ist ein auf einer Rundstrickmaschine gestrickter medizinischer Kompressionsstrumpf zum postoperativen Einsatz bekannt, der aus ein Grundgestrick aus einem elastischen Faden gestrickt ist und am vorderen Zehenende über eine Inspektionsöffnung verfügt, durch die die Zehen eines Patienten für Untersuchungszwecke durchgeführt werden können. Um die Öffnung herum sind über das Grundgestrick vorstehende Rippen angeordnet, welche die Öffnung zusammenziehen.

Die WO 2012/067645 A1 zeigt einen auf einer Rundstrickmaschine gestrickten Socken mit lokalen Kompressionszonen mit axial verlaufenden Kompressionsrippen variabler Länge. Die DE 100 84580 C2 offenbart einen gestrickten Kompressionshandschuh mit wenigstens einem angepassten Bereich, der in das Gestrick des Handschuhs eingearbeitet ist und eine Dehnbarkeit hat, die von derjenigen benachbarter Teile des gestrickten Kompressionshandschuhs abweicht.

Aus der EP 1 679 012 B1 ist ein nahtloser Kompressionshandschuh bekannt, der auf einer Flachstrickmaschine mit einem vorderen und einem hinteren Nadelbett gestrickt wird. Der Kompressionshandschuh besteht aus einem Grundgestrick, das entweder von einer Fingerspitze zur Öffnung des Handschuhs oder von der Öffnung zu der Fingerspitze gestrickt wird, wobei das Grundgestrick auf einer Rippstrick-Strukturbasis unter Verwendung eines Stretch-Elastikgarns, welches in einem gespannten Zustand in das Grundgestrick eingelegt wird, gestrickt ist. Bevorzugt erfolgt das Stricken von der Fingerspitze zur Öffnung hin, wobei das Stretch-Elastikgarn wenigstens an einer Stelle, an der das Stricken des Fingers beginnt, sowie an einem Fingergabelungsteil geknotet wird, um einen Abwurf des Stretch-Elastikgarns zu verhindern. Dabei wird ein vorderer Teil und ein Rückteil jedes Fingers durch ein Rippstricken hoher Maschendichte gestrickt, so dass an jedem Fingergabelungsteil ein flacher Zwickel gebildet wird. Der bekannte Kompressionshandschuh zeichnet sich durch eine hohe Stützwirkung (d.h., durch einen hohen Kompressionsdruck, den der Handschuh auf die Hand eines Trägers ausübt) aus, und kann daher für die Kompressionstherapie, beispielsweise für die Behandlung von Lymphinsuffizienzen an der Hand, eingesetzt werden. Aufgrund seiner hohen Stützleitung, die im Wesentlichen durch das in das Grundgestrick eingebundene Stretch-Elastikgarn bewirkt wird, ist der bekannte Kompressionshandschuh schwer anzuziehen. Insbesondere im Bereich der Finger ist es für einen an einer Lymphinsuffizienz leidenden Patienten schwierig, den engen, komprimierenden Handschuh über die Finger zu ziehen.

Hiervon ausgehend liegt der Erfindung die Aufgabe zugrunde, einen auf einer Flachstrickmaschine nahtlos strickbaren Kompressionsartikel, wie z.B. einen Kompressionshandschuh, einen Kompressionsstrumpf oder einen Kompressionsfüßling, bereit zu stellen, der wenigstens eine schlauch- oder taschenförmige Aufnahme für wenigstens einen Finger oder wenigstens einen Zeh eines Trägers des Kompressionsartikels aufweist und sich einfacher anziehen lässt und dennoch eine hohe Stützleistung aufweist und einen hohen und gleichmäßigen Kompressionsdruck auf eine Körperextremität des Trägers, an der der Kompressionsartikel angelegt wird, ausüben kann.

Diese Aufgabe wird mit einem nahtlosen Kompressionsartikel mit den Merkmalen des Anspruchs 1 gelöst. Bevorzugte Ausführungsformen des Kompressionsartikels sind in den abhängigen Ansprüchen aufgezeigt.

Der nahtlos auf einer Flachstrickmaschine mit einem vorderen und einem hinteren Nadelbett strickabre Kompressionsartikel gemäß der Erfindung umfasst ein Grundgestrick aus wenigstens einem Strickfaden und wenigstens einem darin eingelegten oder eingestrickten elastischen Schussfaden und weist wenigstens eine schlauch- oder taschenförmige und sich entlang einer Längsrichtung erstreckende Aufnahme für wenigstens einen Finger oder wenigstens einen Zeh eines Trägers des Kompressionsartikels auf und ist an einer sich zumindest im Wesentlichen in der Längsrichtung erstreckenden Körperextremität des Trägers, beispielsweise eine Hand- oder einen Fuß, anlegbar. Das Anziehen des Kompressionsartikels wird gemäß der Erfindung dadurch erleichtert, dass die oder jede Aufnahme eine Mehrzahl von rippenförmigen Erhebungen umfasst, die zumindest im Wesentlichen parallel zueinander und entlang der Längsrichtung der jeweiligen Aufnahme verlaufen und durch Flottungen und/oder Fangmaschen des Strickfadens gebildet sind.

Es hat sich gezeigt, dass die bekannten nahtlosen Kompressionsartikel, insbesondere der eingangs beschriebene Kompressionshandschuh, die auf einer Flachstrickmaschine unter Einbindung eines elastischen Schussfadens in ein Grundgestrick gestrickt werden, deshalb schwierig anzuziehen sind, weil sie eine hohe Längszügigkeit, d.h., eine hohe Dehnbarkeit in Längsrichtung, aufweisen. Die Längszügigkeit wird dabei einerseits durch die Ausbildung des Grundgestricks und andererseits durch die Verwendung eines elastischen Strickfadens, aus dem das Grundgestrick gestrickt wird, hervorgerufen. Die Verwendung eines elastischen Strickfadens ist für die Erzeugung einer hohen Stützleistung bzw. einer hohen Kompressionswirkung zweckdienlich.

Der Erfindung liegt die Erkenntnis zugrunde, dass die hohe Längszügigkeit der bekannten, nahtlos auf einer Flachstrickmaschine gestrickten Kompressionsartikel reduziert werden kann, wenn zumindest im Bereich der oder jeder Aufnahme des Kompressionsartikels, die zur Aufnahme wenigstens eines Fingers oder Zehs des Trägers vorgesehen ist, eine Mehrzahl von rippenförmigen Erhebungen, die parallel zueinander verlaufen und sich entlang der Längsrichtung der jeweiligen Aufnahme erstrecken, in das Grundgestrick eingestrickt werden. Die rippenförmigen Erhebungen stehen dabei bevorzugt auf der Außenseite über dem Grundgestrick vor. Die Rippenförmigen Erhebungen, die über dem Grundgestrick vorstehen, sind dabei zu unterscheiden von den Rippen, die sich beim Stricken des Grundgestricks auf Basis einer Rippstricktechnik bzw. eines Rippenstrickmusters ergeben und sich über die gesamte Fläche des in Form eines Rippenstrickmusters gestrickten Grundgestricks erstrecken. Das Grundgestrick des erfindungsgemäßen Kompressionsartikels kann demgemäß ebenfalls mit einer Rippstrick-Technik bzw. mit einem Rippenstrickmuster gestrickt werden, weist dann allerdings zusätzlich zu dem sich durch die Rippstricktechnik ausbildenden Rippenmuster des Grundgestricks, das sich über die gesamte Oberfläche des Grundgestricks erstreckt, zusätzlich noch im Bereich der oder jeder Aufnahme für wenigstens einen Finger oder Zeh eine Mehrzahl von rippenförmigen Erhebungen auf, die, bevorzugt an der Außenseite, über dem Grundgestrick vorstehen.

Der erfindungsgemäße Kompressionsartikel besteht zweckmäßig aus einem Grundgestrick mit einer beim Tragen an einer Körperextremität dieser zugewandten Innenseite und einer der Innenseite gegenüberliegenden Außenseite. Das Grundgestrick ist dabei entlang einer Maschenstäbchenrichtung gestrickt und weist quer zur Maschenstäbchenrichtung verlaufende Maschenreihen auf. Beim Anlegen des Kompressionsartikels an eine Körperextremität eines Trägers verläuft dabei die Maschenstäbchenrichtung zumindest im Wesentlichen entlang der Längsrichtung der Körperextremität. Insbesondere verläuft die Maschenstäbchenrichtung im Bereich der oder jeder Aufnahme des Kompressionsartikels, der zur Aufnahme wenigstens eines Fingers oder Zehs des Trägers vorgesehen ist, entlang der Längsrichtung der jeweiligen Aufnahme.

Eine Aufnahme kann dabei, je nach Ausdehnung der Aufnahme quer zur Längsrichtung, zur Aufnahme nur eines Fingers oder Zehs oder auch zur Aufnahme mehrerer Finger bzw. Zehen vorgesehen sein.

Die Ausbildung der rippenförmigen Erhebungen in der Weise, dass Sie an der Außenseite des Kompressionsartikels über dem Grundgestrick vorstehen, erleichtert zum einen die Konfektionierung des Kompressionsartikels auf einer Flachstrickmaschine und verhindert außerdem, dass ein Träger des Kompressionsartikels beim Anziehen mit einem Fingernagel oder einem Zehennagel an den rippenförmigen Erhebungen hängen bleiben könnte.

Es hat sich gezeigt, dass die Anziehbarkeit des erfindungsgemäßen Kompressionsartikels bereits durch Ausbildung von wenigstens zwei rippenförmigen Erhebungen an jeder schlauch- oder taschenförmigen Aufnahme verbessert wird. Je nach Umfang der schlauch- oder taschenförmigen Aufnahmen können bis zu 30 rippenförmige Erhebungen an jeder Aufnahme eingestrickt werden. Bevorzugt weist jede Aufnahme zwischen 3 und 7 rippenförmige Erhebungen auf.

Die rippenförmigen Erhebungen erstrecken sich in jeder Maschenreihe des Grundgestricks bevorzugt über mindestens drei Maschen.

Zur Erzielung einer hohen Kompressionswirkung ist es vorteilhaft, wenn in das aus einem Strickfaden gestrickte Grundgestrick ein elastischer Schussfaden eingelegt bzw. eingebunden wird. Eine besonders hohe Kompressionswirkung kann dabei erzielt werden, wenn in jeder Maschenreihe des Grundgestricks ein elastischer Schussfaden eingelegt bzw. eingebunden wird.

Die sich entlang der Längsrichtung der jeweiligen Aufnahme für einen Finger oder einen Zeh erstreckenden Erhebungen sind in einem in Maschenreihenrichtung liegenden, vorgegebenen Abstand zueinander in dem Grundgestrick angeordnet. Bevorzugt liegen zwischen zwei in einer Maschenreihe aufeinanderfolgende Erhebungen wenigstens zwei Maschen des Strickfadens, aus dem das Grundgestrick gestrickt ist. Der Abstand zwischen benachbarten Erhebungen in einer Maschenreihe kann sich jedoch auch über mehr als zwei Maschen des Grundgestricks, bspw. über fünf bis zehn Maschen, erstrecken.

Wenn eine geringere Kompressionswirkung benötigt wird, kann auch nur in jeder zweiten Maschenreihe ein elastischer Schussfaden vorgesehen sein. Weiterhin ist es möglich, in druckgeminderten Bereichen des Grundgestricks, die sich über mehr als zwei in Maschenstäbchenrichtung aufeinanderfolgende Maschenreihen erstrecken, in mehreren, aufeinander folgenden Maschenreihen keinen Schussfaden einzubringen.

Die Ausbildung der rippenförmigen Erhebungen in dem Grundgestrick erfolgt durch Flottungen des Strickfadens des Grundgestricks und/oder durch Ausbildung von Fanghenkeln. Dabei kann in einem bevorzugten Ausführungsbeispiel des Kompressionsartikels, in dem in das Grundgestrick ein Schussfaden eingebunden wird, der Schussfaden zwischen zwei in Richtung der Maschenreihen aufeinanderfolgende Erhebungen als Fang in das Grundgestrick eingebunden sein. Bevorzugt wird der Schussfaden dabei zwischen zwei in Maschenreihenrichtung aufeinanderfolgende Erhebungen in jeder zweiten Masche des Grundgestricks als Fang in das Grundgestrick eingebunden.

Eine Abgrenzung der über das Grundgestrick vorstehenden Erhebungen kann durch Ausbildung von zwei in den Maschenreihen aufeinanderfolgenden Fangmaschen des Schussfadens in den Randbereichen der Erhebungen ausgebildet werden.

Zur Minimierung der Längszügigkeit der zur Aufnahme wenigstens eines Fingers oder Zehs vorgesehenen Aufnahmen des Kompressionsartikels ist es zweckmäßig, wenn sich die Erhebungen in Längsrichtung der oder jeder Aufnahme zumindest im Wesentlichen über die gesamte Ausdehnung der Aufnahme in Längsrichtung erstrecken.

Um eine möglichst hohe Kompressionswirkung des Kompressionsartikels außerhalb des Bereichs, in dem sich die Aufnahmen befinden, zu gewährleisten, ist es zweckmäßig, wenn in den Bereichen außerhalb der Aufnahmen keine rippenförmigen Erhebungen im Sinne der Erfindung vorhanden sind. Insbesondere bei Maßanfertigungen von Kompressionsartikeln gemäß der Erfindung ist es darüber hinaus einfacher, den Kompressionsartikel zu konfektionieren, wenn außerhalb der Aufnahmen keine rippenförmigen Erhebungen vorhanden sind.

Der erfindungsgemäße Kompressionsartikel kann auf einer Flachstrickmaschine mit einem vorderen und einem hinteren Nadelbett gestrickt werden, wobei das Grundgestrick des Kompressionsartikels dadurch eine vordere Lage, die auf dem vorderen Nadelbett gestrickt wird, und eine hintere Lage, die auf dem hinteren Nadelbett gestrickt wird, erhält und dadurch schlauchförmig ausgebildet wird. Die vordere und die hintere Lage des Kompressionsartikels sind dabei nahtlos miteinander verbunden, d.h. miteinander verstrickt. Dabei enthält die vordere und die hintere Lage des Grundgestricks jeweils einen Strickfaden, wobei der Strickfaden zur Ausbildung der rippenförmigen Erhebungen Flottungen bildet und die Stellen, an denen der Strickfaden flott liegt, in jeweils in Maschenstäbchenrichtung aufeinanderfolgenden Maschenreihen um wenigstens eine Masche, bevorzugt um eine oder zwei Maschen, versetzt zueinander angeordnet sind. Durch die Anzahl der Maschen, um die die Versetzung der Flottungen des Strickfadens erfolgt, kann die Breite der rippenförmigen Erhebungen (also deren Ausdehnung in Maschenreihenrichtung) definiert werden.

Diese und weitere Vorteile sowie zweckmäßige Merkmale der Erfindung ergeben sich aus den nachfolgend unter Bezugnahme auf die begleitenden Zeichnungen näher beschriebenen Ausführungsbespielen. Die Zeichnungen zeigen:
- **Fig. 1:**: Schematische Darstellung eines Strickbilds einer ersten Ausführungsform eines Gestricks, aus dem zumindest ein Teilbereich des erfindungsgemäßen Kompressionsartikels hergestellt werden kann;
- **Fig. 2:**: Darstellung der Außenseite (Figur 2a) und der Innenseite (Fig. 2b) des für die Herstellung eines erfindungsgemäßen Kompressionsartikels bevorzugten Gestricks;
- **Fig. 3:**: Darstellung von als Kompressionsärmel ausgebildeten Kompressionsartikeln gemäß der Erfindung, wobei in Figur 3a eine erste Ausführungsform eines Kompressionsärmels gezeigt ist, in dem lediglich im Bereich der Fingeraufnahmen rippenförmige Erhebungen vorgesehen sind, und in Figur 3b eine zweite Ausführungsform eines Kompressionsärmels gezeigt ist, bei dem über die gesamte Länge des Ärmels rippenförmige Erhebungen gemäß der Erfindung vorgesehen sind;
- **Fig. 4:**: Darstellung von als Kompressionshandschuh ausgebildeten Kompressionsartikeln gemäß der Erfindung, wobei in Figur 3a eine erste Ausführungsform eines Kompressionshandschuhs gezeigt ist, in dem lediglich im Bereich der Fingeraufnahmen rippenförmige Erhebungen vorgesehen sind, und in Figur 3b eine zweite Ausführungsform eines Kompressionshandschuhs gezeigt ist, bei dem über die gesamte Länge des Ärmels rippenförmige Erhebungen gemäß der Erfindung vorgesehen sind;
- **Fig. 5:**: Darstellung eines als Kompressionsstrumpf (Fig. 5a) und als Füßling (Fig. 5b) ausgebildeten Ausführungsbeispiels eines erfindungsgemäßen Kompressionsartikels, wobei sich jeweils im Bereich der Zehenaufnahmen des Kompressionsstrumpfs der Figur 5a und des Füßlings der Figur 5b rippenförmige Erhebungen in Längsrichtung der jeweiligen Zehenaufnahme erstrecken;
- **Fig. 6:**: Schematische Darstellung eines Strickbilds einer weiteren Ausführungsform eines Gestricks, aus dem zumindest ein Teilbereich des erfindungsgemäßen Kompressionsartikels hergestellt werden kann.

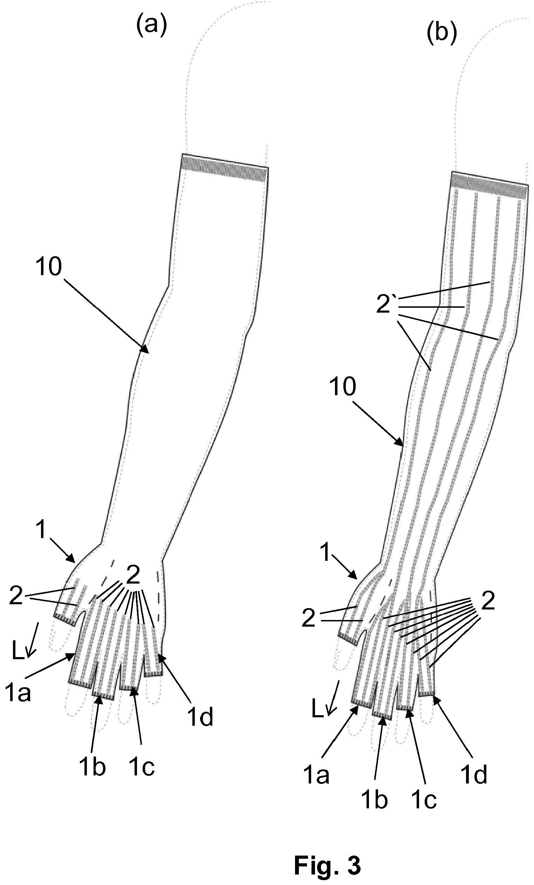

Die beispielhaft in Figur 3 dargestellten und als Kompressionsärmel 10 ausgebildeten Ausführungsbeispiele eines erfindungsgemäßen Kompressionsartikels weisen fünf Aufnahmen 1, 1a, 1b, 1c, 1d zur Aufnahme der fünf Finger einer Hand eines Trägers der Kompressionsärmel auf, wobei eine Aufnahme 1 zur Aufnahme des Daumens und die anderen Aufnahmen 1a - 1d zur Aufnahme der übrigen Finger der Hand vorgesehen sind. Die Aufnahmen, die im Folgenden zusammen jeweils mit Bezugsziffer 1 gekennzeichnet sind, erstrecken sich dabei entlang einer Längsrichtung L, die der Längsrichtung des jeweiligen Fingers der Hand entspricht. Bei dem hier zeichnerisch dargestellten Ausführungsbeispiel sind die Fingeraufnahmen 1 jeweils offen und weisen am offenen Ende ein Bündchen auf. Im Rahmen der Erfindung können die Aufnahmen 1 jedoch auch geschlossen, d.h., mit einer geschlossenen Fingerspitze, ausgebildet sein. Bei dieser Ausführungsform entfällt das Bündchen.

In den beiden in Figur 3 gezeigten Ausführungsbeispielen eines erfindungsgemäßen Kompressionsartikels in Form eines Kompressionsärmels sind im Bereich der Aufnahmen 1 jeweils mehreren, rippenförmige Erhebungen 2 vorgesehen, die zumindest im Wesentlichen parallel zueinander verlaufen und sich entlang der Längsrichtung L der jeweiligen Aufnahme 1 erstrecken. An jeder Aufnahme 1 ist dabei eine Mehrzahl von rippenförmigen Erhebungen 2 angeordnet, wobei sich die rippenförmigen Erhebungen 2 zumindest im Wesentlichen über die gesamte Ausdehnung der jeweiligen Aufnahme 1 in ihrer Längsrichtung L erstrecken. In dem in Figur 3a gezeigten Ausführungsbeispiel eines Kompressionsärmels sind außerhalb der Bereiche der Aufnahmen 1 keine Erhebungen 2 vorgesehen. Bei dem in Figur 3b gezeigten Ausführungsbeispiel eines Kompressionsärmels sind dagegen auch in den Bereichen außerhalb der Aufnahmen 1 rippenförmige Erhebungen 2' vorgesehen, die sich zumindest im Wesentlichen entlang der Längsrichtung des Ärmels erstrecken und wenigstens annähernd parallel zueinander verlaufen.

Die rippenförmigen Erhebungen 2, 2' vermindern dabei die Längszügigkeit des Kompressionsartikels im Bereich der Aufnahmen 1 bzw. in dem gesamten Bereich des Kompressionsartikels und erleichtern dadurch das Anziehen des Kompressionsärmels, insbesondere das Überziehen der Aufnahmen 1 über die Finger der Hand des Trägers bzw. über die gesamte Hand und den Arm des Trägers.

Die in Figur 3 gezeigten Kompressionsärmel sind, wie alle anderen Ausführungsformen der erfindungsgemäßen Kompressionsartikel, nahtlos auf einer Flachstrickmaschine mit einem vorderen und einem gegenüberliegenden hinteren Nadelbett gestrickt, wobei die schlauch- oder taschenförmigen Aufnahmen 1, die zur Aufnahme wenigstens eines Fingers oder eines Zehs des Trägers vorgesehen sind und sich entlang der Längsrichtung L erstrecken, nahtlos in dem Kompressionsartikel eingestrickt sind.

Das Gestrick, aus dem die erfindungsgemäßen Kompressionsartikel hergestellt werden können, umfasst ein Grundgestrick aus einem Strickfaden mit einer beim Tragen des Kompressionsartikels an einer Körperextremität dieser zugewandten Innenseite und einer der Innenseite gegenüberliegenden Außenseite, wobei das Grundgestrick entlang einer Maschenstäbchenrichtung gestrickt ist und sich quer zu Maschenstäbchenrichtung verlaufende Maschenreihen aufweist.

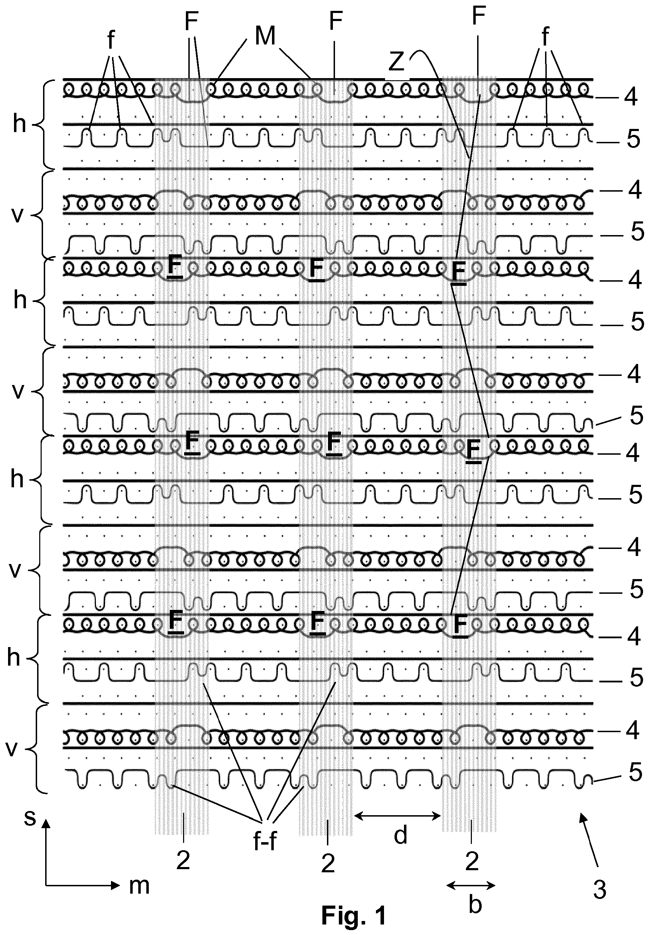

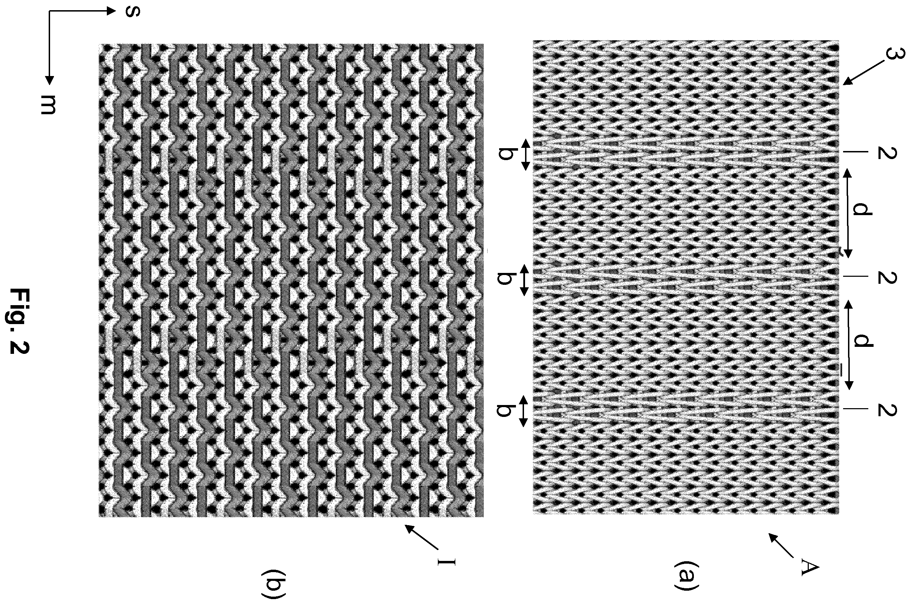

In den Figuren 1 und 2 ist ein bevorzugtes Gestrick dargestellt, aus dem zumindest die Bereiche der Aufnahmen 1 der erfindungsgemäßen Kompressionsartikel hergestellt werden können.

Aus dem Strickbild von Figur 1 ist ersichtlich, dass das Gestrick eine vordere Lage v, die auf dem vorderen Nadelbett der Flachstrickmaschine gestrickt wird, und eine hintere Lage h, die auf dem hinteren Nadelbett gestrickt wird, enthält. In Figur 1 sind die Maschenreihen der vorderen Lage v und der hinteren Lage h entlang der Maschenstäbchenrichtung s übereinander dargestellt. Aus Figur 1 ist die Ausbildung des Grundgestricks der vorderen Lage v und der hinteren Lage h mit den in den Maschenreihen m verlaufenden Maschen M des Grundgestricks ersichtlich.

Das Grundgestrick ist dabei aus einem Strickfaden 4 gebildet, der beispielsweise in einem Rechts-Links-Gestrick oder in einer 1:1-Bindung zur Ausbildung des Grundgestricks auf der Flachstrickmaschine verstrickt wird. In dem in Figur 1 gezeigten Ausführungsbeispiel ist das Grundgestrick als Rechts-Links-Gestrick ausgebildet. Bei dem Strickfaden 4 kann es sich um einen elastischen oder auch um einen zumindest weitgehend unelastischen Strickfaden handeln. Für die Ausbildung einer hohen Kompressionswirkung ist die Verwendung eines elastischen Strickfadens zu bevorzugen. Wenn ein elastischer Strickfaden 4 eingesetzt wird, kann es sich beispielsweise um ein Umwindegarn mit einem elastischen Kernfaden handeln.

Bei dem in Figur 1 gezeigten bevorzugten Ausführungsbeispiel eines für die Herstellung der erfindungsgemäßen Kompressionsartikel geeigneten Gestricks ist in jeder Maschenreihe m des aus dem Strickfaden 4 gebildeten Grundgestricks ein elastischer Schussfaden 5 eingebunden. Bei dem elastischen Schussfaden 5 kann es sich beispielsweise um ein Umwindegarn mit einem hochelastischen Kernfaden oder um einen Elastan- oder Gummifaden handeln. Zweckmäßig weist der Kernfaden oder der gesamte Schussfaden eine Dicke im Bereich von 200 bis 1500 dtex auf. Der Schussfaden 5 ist dabei in der Regel (wesentlich) dicker als der Strickfaden 4.

Die rippenförmigen Erhebungen 2, die zumindest im Bereich der Aufnahme 1 des erfindungsgemäßen Kompressionsartikels vorgesehen sind, werden durch Flottungen F des Strickfadens 4 in den Maschenreihen m des Grundgestricks ausgebildet. In dem Strickbild der Figur 1 sind die zumindest im Wesentlichen parallel zueinander und in Maschenreihenrichtung in einem vorgegebenen Abstand d zueinander angeordneten Erhebungen 2 durch ein graues Linienfeld dargestellt. Die Ausdehnung der Erhebungen 2 in Maschenreihenrichtung m, also deren Breite b, ergibt sich dabei durch die Lage der Flottungen F des Strickfadens 4, die in dem Strickbild der Figur 1 jeweils mit Bezugzeichen F dargestellt sind. In jeweils in Maschenstäbchenrichtung s aufeinanderfolgenden (bzw. übereinanderliegenden) Maschenreihen m der vorderen Lage v und der hinteren Lage h sind die Stellen, an denen der Strickfaden 4 Flottungen F bildet, um wenigstens eine Masche versetzt zueinander angeordnet. Bei dem in Figur 1 dargestellten Strickbild sind die Stellen, an denen der Strickfaden 4 in der vorderen Lage v bzw. der hinteren Lage h Flottungen F bildet, jeweils um eine Masche versetzt zueinander angeordnet. Eine Verbindung Z der Flottungsstellen F in der vorderen Lage v bzw. der hinteren Lage h bildet daher eine um jeweils eine Masche versetzte Zick-Zack-Linie und die Ränder dieser Zick-Zack-Linie in den Maschenreihen m stellen die seitlichen Begrenzungen der Erhebungen 2 dar.

In Maschenreihenrichtung m zwischen zwei aufeinanderfolgende Erhebungen 2 ist eine vorgegebene Anzahl von Maschen M des Strickfaden 4 ausgebildet. Die Anzahl der Maschen M, die sich zwischen zwei in Maschenreihenrichtung m benachbarten Erhebungen 2 befinden, legt den Abstand d der zueinander benachbarten Erhebungen 2 in Maschenreihenrichtung m fest. Bevorzugt liegen zwischen zwei in Maschenreihenrichtung benachbarten Erhebungen 2 wenigstens zwei und bevorzugt mehr als fünf Maschen M. Bei dem in Figur 1 gezeigten Ausführungsbeispiel liegen sechs Maschen M des Strickfadens 4 zwischen in Maschenreihenrichtung m benachbarten Erhebungen 2.

Wie aus dem Strickbild der Figur 1 ersichtlich, ist in jeder Maschenreihe m ein Schussfaden 5 eingebunden. Der Schussfaden 5 bildet dabei Fangmaschen f. Insbesondere in den Randbereichen der Erhebungen 2 bildet der Schussfaden in den Maschenreihen m zwei unmittelbar aufeinanderfolgende Fangmaschen f-f. In den Bereichen zwischen zwei in einer Maschenreihe benachbarten Erhebungen 2 ist der Schussfaden 5 abwechselnd als Fangmasche f und flott liegend in dem durch den Strickfaden 4 gebildeten Grundgestrick eingelegt. Durch die Ausbildung einer doppelten Fangmasche f-f im Randbereich der Erhebungen 2 wird das aus dem Strickfaden 4 gebildete Grundgestrick im Bereich der Erhebungen 2, in dem der Strickfaden 4 eine Flottung F bildet, zusammengezogen, so dass sich eine über den übrigen Bereich des Grundgestricks vorstehende Erhebung 2 ausbildet.

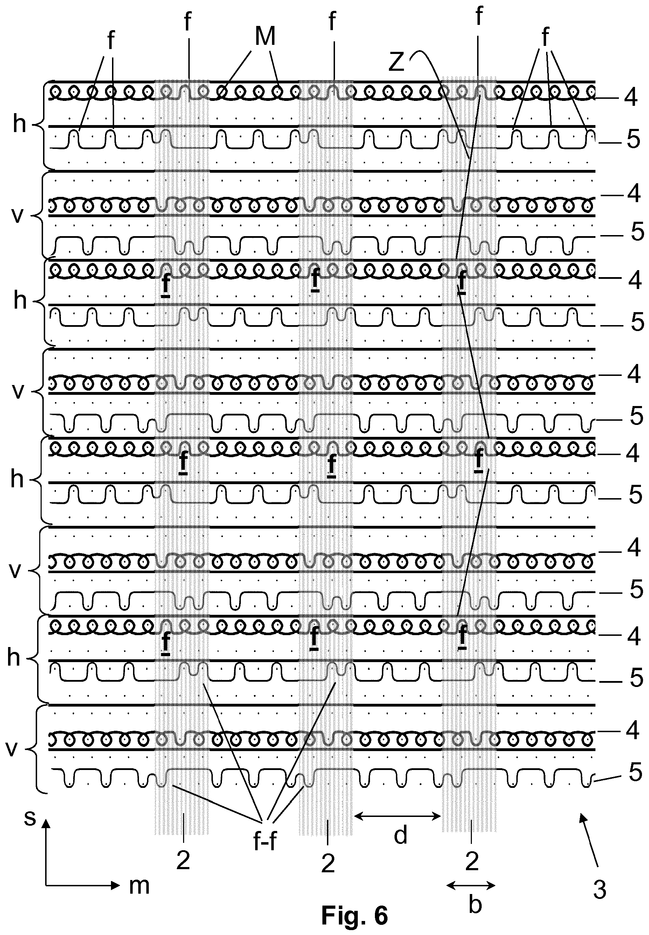

In Figur 6 ist eine weitere Ausführungsform eines Gestricks dargestellt, aus dem zumindest die Bereiche der Aufnahmen 1 der erfindungsgemäßen Kompressionsartikel hergestellt werden können. Diese Ausführungsform unterscheidet sich von dem in Figur 1 gezeigten Gestrick dadurch, dass die Erhebungen 2 durch Ausbildung von Fanghenkeln f durch den Strickfaden 4 ausgebildet sind. Hierfür sind an den Stellen, an denen der Strickfaden 4 bei der Ausführungsform der Figur 1 Flottungen F bildet, an Stelle der Flottungen Fanghenkel f ausgebildet. An den Stellen der Fanghenkel f bildet der Strickfaden 4 - ebenso wie bei den Flottungsstellen F der Ausführungsform von Figur 1 - keine Maschen, wodurch die Erhebungen 2 entstehen.

Bevorzugt wird ein erfindungsgemäßer Kompressionsartikel aus dem in Figur 1 oder dem in Figur 6 schematisch dargestellten Gestrick so gestrickt, dass die über dem Grundgestrick vorstehenden rippenförmigen Erhebungen 2 auf der Außenseite des Kompressionsartikels, der beim Anlegen an einer Körperextremität dieser abgewandt ist, zu liegen kommen.

In Figur 2 ist die Außenseite A und die Innenseite I des Gestricks von Figur 1 dargestellt, wobei Figur 2a die Außenseite A und Figur 2b die Innenseite I zeigt. Aus Figur 2a sind die Erhebungen 2, die sich in Längsrichtung L und in Maschenreihenrichtung m im Abstand d zueinander erstrecken und eine Breite b aufweisen, ersichtlich. Die Erhebungen 2 stehen dabei über das Grundgestrick vor und bilden dadurch rippenförmige Erhebungen 2. Auf der Innenseite I des Gestricks kommen die Erhebungen 2 nicht zum Vorschein. Dies hat den Vorteil, dass die Erhebungen 2 beim Anziehen des Kompressionsartikels nicht stören und der Träger des Kompressionsartikels insbesondere nicht mit einem Finger- oder Zehennagel an den Erhebungen 2 hängen bleiben kann.

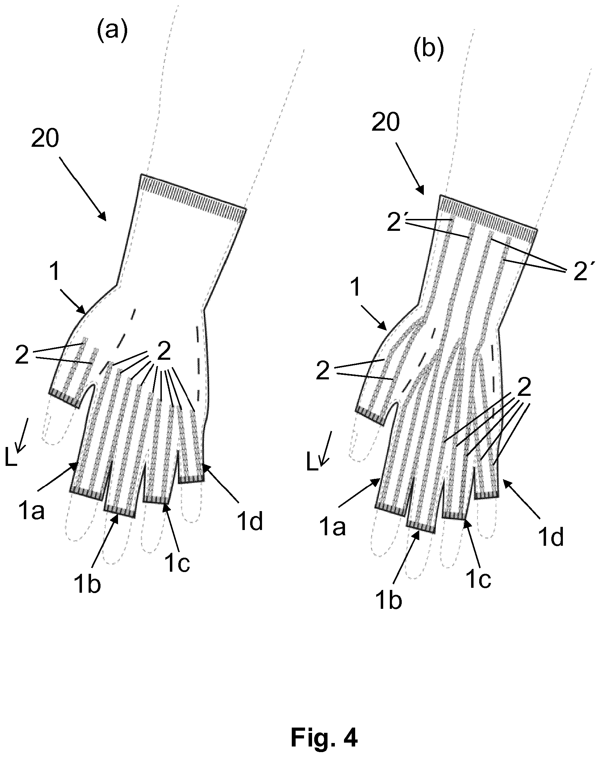

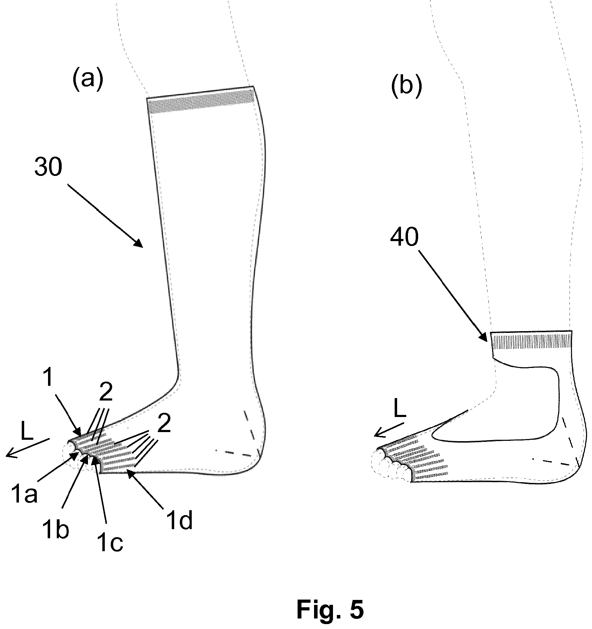

In den Figuren 4 und 5 sind weitere Ausführungsbeispiele von Kompressionsartikeln gemäß der Erfindung gezeigt. Figur 4 zeigt dabei zwei als Kompressionshandschuhe 20 ausgebildete Kompressionsartikel, wobei bei dem in Figur 4a gezeigten Kompressionshandschuh lediglich im Bereich der Aufnahmen 1 für die Finger des Trägers rippenförmige Erhebungen 2 vorgesehen sind, wohingegen bei dem in Figur 4b gezeigten Ausführungsbeispiel eines Kompressionshandschuhs auch in dem Bereich außerhalb der Aufnahmen 1 rippenförmige Erhebungen 2' angeordnet sind.

In Figur 5 sind Kompressionsartikel gern, der Erfindung zum Anlegen an ein Bein bzw. einen Fuß eines Trägers gezeigt. In Figur 5a ist ein Kompressionsstrumpf 30 dargestellt, der an einem Unterschenkel eines Trägers angelegt werden kann. Der Unterschenkel-Kompressionsstrumpf weist dabei Aufnahmen 1, 1a, 1b, 1c, 1d zur Aufnahme der Zehen des Trägers auf. Die Aufnahmen 1, 1a-1d sind dabei jeweils offen ausgebildet und enthalten am offenen Ende ein Bündchen. Es ist jedoch auch möglich, die Zehenaufnahmen 1, 1a-1d geschlossen auszubilden, so dass die Zehen des Trägers vollständig in der jeweils vorgesehenen Aufnahme 1, 1a-1d aufgenommen werden können. Wie in den Ausführungsführungsbeispielen der Figuren 3 und 4 sind auch bei den in Figur 5 gezeigten Ausführungsbeispielen im Bereich der Aufnahmen 1 für die Zehen rippenförmige Erhebungen 2 angeordnet, die sich jeweils in Längsrichtung L der jeweiligen Aufnahme 1 erstrecken und quer zur Längsrichtung in einem vorgegebenen Abstand d zueinander sind und parallel zueinander verlaufen. Außerhalb der Aufnahmen 1 sind dabei keine Erhebungen 2 vorgesehen. Das in Figur 5b gezeigte Ausführungsbeispiel zeigt einen Füßling 40 mit Aufnahmen 1 zur Aufnahme der Zehen des Trägers. Der Füßling weist im Bereich des Rists und des Sprunggelenks eine Ausnehmung auf. Diese Ausnehmung kann auch weggelassen werden, so dass der Fuß des Trägers, einschl. des Rists und des Sprunggelenks, vollständig in Form einer Socke von dem Kompressionsartikel umfasst wird.

Es ist für einen Fachmann leicht ersichtlich, dass weitere Formen von Kompressionsartikeln mit wenigstens einer Aufnahme für mindestens einen Finger oder Zeh, bspw. Socken oder Fingerlinge, gemäß der Erfindung ausgebildet werden können.

## Patentansprüche

1. Nahtloser Kompressionsartikel, der ein Grundgestrick (3) aus wenigstens einem Strickfaden (4) und wenigstens einem darin eingelegten oder eingestrickten elastischen Schussfaden (5) umfasst sowie wenigstens eine schlauch- oder taschenförmige und sich entlang einer Längsrichtung (L) erstreckende Aufnahme (1) für wenigstens einen Finger (F) oder wenigstens einen Zeh (Z) eines Trägers des Kompressionsartikels aufweist und an einer sich in Längsrichtung (L) erstreckenden Körperextremität des Trägers anlegbar ist, wobei die oder jede Aufnahme (1) eine Mehrzahl von rippenförmigen Erhebungen (2) umfasst, die zumindest im Wesentlichen parallel zueinander und entlang der Längsrichtung (L) der jeweiligen Aufnahme (1) verlaufen, **dadurch gekennzeichnet, dass** die rippenförmigen Erhebungen (2) durch Flottungen (F) und/oder Fangmaschen (f) des Strickfadens (4) gebildet sind.

2. Kompressionsartikel nach Anspruch 1, **dadurch gekennzeichnet, dass** das Grundgestrick (3) eine beim Tragen der Körperextremität zugewandte Innenseite (I) und eine dieser gegenüber liegenden Außenseite (A) aufweist, wobei das Grundgestrick (3) pentlang einer sich zumindest im Wesentlichen in Längsrichtung (L) der oder jeder Aufnahme (1) erstreckenden Maschenstäbchenrichtung (s) gestrickt ist und sich quer zur Maschenstäbchenrichtung (s) verlaufende Maschenreihen (m) aufweist, wobei die rippenförmigen Erhebungen (2) an der Außenseite (A) über dem Grundgestrick (3) vorstehen.

3. Kompressionsartikel nach einem der voranstehenden Ansprüche, **dadurch gekennzeichnet, dass** an jeder Aufnahme (1) zwischen zwei und 30 Erhebungen (2) angeordnet sind und/oder dass sich die Erhebungen (2) in einer Maschenreihe (m) des Grundgestricks (3) über mindestens zwei Maschen erstrecken.

4. Kompressionsartikel nach einem der voranstehenden Ansprüche, **dadurch gekennzeichnet, dass** benachbarte Erhebungen (2) parallel und in einem vorgegebenen Abstand (d) zueinander verlaufen, wobei zwischen zwei in einer Maschenreihe (m) aufeinanderfolgende Erhebungen (2) wenigstens zwei Maschen des Strickfadens (4) liegen.

5. Kompressionsartikel nach einem der voranstehenden Ansprüche, **dadurch gekennzeichnet, dass** in jeder oder in jeder zweiten Maschenreihe (m) des Grundgestricks (3) ein Schussfaden (5) verläuft.

6. Kompressionsartikel nach einem der voranstehenden Ansprüche, **dadurch gekennzeichnet, dass** in druckgeminderten Bereichen des Grundgestricks (3) mehrere in Längsrichtung (L) aufeinanderfolgende Maschenreihen (m) vorhanden sind, in denen kein Schussfaden verläuft.

7. Kompressionsartikel nach einem der voranstehenden Ansprüche, **dadurch gekennzeichnet, dass** der Schussfaden (5) zwischen zwei in Richtung der Maschenreihen (m) aufeinanderfolgende Erhebungen (2) in jeder oder jeder zweiten Masche als Fangmasche (f) in das Grundgestrick (3) des Strickfadens (4) eingebunden ist.

8. Kompressionsartikel nach einem der voranstehenden Ansprüche, **dadurch gekennzeichnet, dass** der Schussfaden (5) in einem Randbereich der Erhebungen (2) zwei in einer Maschenreihe (m) aufeinanderfolgende Fangmaschen (f-f) bildet.

9. Kompressionsartikel nach einem der voranstehenden Ansprüche, **dadurch gekennzeichnet, dass** sich die Erhebungen (2) in Längsrichtung (L) der oder jeder Aufnahme (1) zumindest im Wesentlichen über die gesamte Ausdehnung der jeweiligen Aufnahme (1) erstrecken, wobei in den Bereichen des Kompressionsartikels außerhalb der Aufnahmen (1) keine Erhebungen (2) vorhanden sind.

10. Kompressionsartikel nach einem der voranstehenden Ansprüche, **dadurch gekennzeichnet, dass** das Grundgestrick (3) aus einem Gestrick mit einer vorderen Lage (v) und einer der vorderen Lage (v) gegenüberliegenden hinteren Lage (h) gebildet ist.

11. Kompressionsartikel nach Anspruch 10, **dadurch gekennzeichnet, dass** die vordere Lage (v) und die hintere Lage (h) jeweils den Strickfaden (4) enthalten, wobei der Strickfaden (4) zur Ausbildung der rippenförmigen Erhebungen (2) Flottungen (F) oder Fanghenkel (f) bildet und die Stellen, an denen der Strickfaden (4) flott liegt oder Fanghenkel (f) bildet, in jeweils aufeinanderfolgenden Maschenreihen (m) um wenigstens eine Masche, bevorzugt um eine oder zwei Maschen, versetzt zueinander angeordnet sind.

12. Kompressionsartikel nach einem der voranstehenden Ansprüche, **dadurch gekennzeichnet, dass** es sich bei dem elastischen Schussfaden (5) um ein Umwindegarn mit einem hochelastischen Kernfaden, der eine Dicke im Bereich von 200 bis 1500 dtex aufweist, oder um einen Elasthan- oder Gummifaden handelt.

13. Kompressionsartikel nach einem der voranstehenden Ansprüche, **dadurch gekennzeichnet, dass** es sich bei dem Grundgestrick (3) um ein rechts-links-Gestrick handelt und/oder dass das Grundgestrick (3) in einer 1:1-Bindung gestrickt ist.

14. Kompressionsartikel nach einem der voranstehenden Ansprüche, **dadurch gekennzeichnet, dass** es sich bei dem Kompressionsartikel um einen Handschuh handelt, wobei eine Aufnahme (1) zur Aufnahme eines Daumens und/oder wenigstens eine weitere Aufnahme (1a, 1b, 1c, 1d) zur Aufnahme eines Fingers dienen, oder dass es sich bei dem Kompressionsartikel um einen Strumpf handelt, wobei wenigstens eine Aufnahme (1) zur Aufnahme einer Zehe und/oder weitere Aufnahmen (1a, 1b, 1c, 1d) zur Aufnahme weiterer Zehen dienen.

15. Kompressionsartikel nach einem der voranstehenden Ansprüche, **dadurch gekennzeichnet, dass** das Grundgestrick (3) ein vollflächig durchgehendes Rippenmuster und zusätzlich zumindest im Bereich der oder jeder Aufnahme (1) die rippenförmigen Erhebungen (2) aufweist, die über dem Rippenmuster des Grundgestricks (3) vorstehen.

## Claims

1. Seamless compression article comprising a basic knitted fabric (3) of at least one knitting yarn (4) and at least one elastic weft yarn (5) inserted or knitted therein, and having at least one tubular or pocket-shaped receptacle (1) extending along a longitudinal direction (L) for at least one finger (F) or at least one toe (Z) of a wearer of the compression article and adapted to be applied to a body extremity of the wearer extending in the longitudinal direction (L), wherein the or each receptacle (1) comprises a plurality of rib-shaped protrusions (2) extending at least substantially parallel to each other and along the longitudinal direction (L) of the respective receptacle (1), **characterised in that** the rib-shaped protrusions (2) are formed by floats (F) and/or tuck stitches (f) of the knitting yarn (4).

2. Compression article according to claim 1, **characterised in that** the basic knitted fabric (3) has an inner side (I) facing the body extremity when worn and an outer side (A) opposite thereto, the basic knitted fabric (3) being knitted along a wale direction (s) extending at least substantially in the longitudinal direction (L) of the or each receptacle (1) and having wale rows (m) extending transversely of the wale direction (s), the rib-like protrusions (2) on the outer side (A) projecting above the basic knitted fabric (3).

3. Compression article according to one of the preceding claims, **characterised in that** between two and 30 protrusios (2) are arranged on each receptacle (1) and/or **in that** the protrusios (2) extend over at least two stitches in a row of stitches (m) of the basic knitted fabric (3).

4. Compression article according to one of the preceding claims, **characterised in that** adjacent protrusios (2) run parallel to one another and at a predetermined distance (d) from one another, at least two stitches of the knitting yarn (4) being located between two protrusions (2) which follow one another in a row of stitches (m).

5. Compression article according to one of the preceding claims, **characterised in that** a weft yarn (5) runs in each or in every second row of stitches (m) of the basic knitted fabric (3).

6. Compression article according to one of the preceding claims, **characterised in that** in pressure-reduced regions of the basic knitted fabric (3) there are a plurality of rows of stitches (m) which follow one another in the longitudinal direction (L) and in which no weft yarn runs.

7. Compression article according to one of the preceding claims, **characterised in that** the weft yarn (5) is bound into the basic knitted fabric (3) of the knitting yarn (4) as a tuck stitch (f) in every stitch or every second stitch between two successive protrusions (2) in the direction of the rows of stitches (m).

8. Compression article according to one of the preceding claims, **characterised in that** the weft yarn (5) forms two tuck stitches (f-f) following one another in a row of meshes (m) in an edge region of the protrusions (2).

9. Compression article according to any one of the preceding claims, **characterised in that** the protrusions (2) extend in the longitudinal direction (L) of the or each receptacle (1) at least substantially over the entire extent of the respective receptacle (1), wherein no protrusions (2) are present in the regions of the compression article outside the receptacles (1).

10. Compression article according to any one of the preceding claims, **characterised in that** the basic knitted fabric (3) is formed from a knit having a front layer (v) and a rear layer (h) opposite the front layer (v).

11. Compression article according to claim 10, **characterised in that** the front layer (v) and the rear layer (h) each contain the knitting yarn (4), the knitting yarn (4) forming floats (F) or tuck stitches (f) to form the rib-shaped protrusions (2), and the points at which the knitting yarn (4) floats or forms tuck stitches (f) being arranged offset relative to one another by at least one stitch, preferably by one or two stitches, in respective successive rows of stitches (m).

12. Compression article according to any one of the preceding claims, **characterised in that** the elastic weft yarn (5) is a wrap yarn having a highly elastic core yarn having a thickness in the range 200 to 1500 dtex, or a spandex or rubber yarn.

13. Compression article according to one of the preceding claims, **characterised in that** the basic knitted fabric (3) is a right-to-left knitted fabric and/or that the basic knitted fabric (3) is knitted in a 1:1 weave.

14. Compression article according to one of the preceding claims, **characterized in that** the compression article is a glove, wherein one receptacle (1) serves to receive a thumb and/or at least one further receptacle (1a, 1b, 1c, 1d) serves to receive a finger, or **in that** the compression article is a stocking, wherein at least one receptacle (1) serves to receive a toe and/or further receptacles (1a, 1b, 1c, 1d) serve to receive further toes.

15. Compression article according to one of the preceding claims, **characterised in that** the basic knitted fabric (3) has a rib pattern extending over the entire surface and additionally, at least in the region of the or each receptacle (1), the rib-shaped protrusions (2) which project above the rib pattern of the basic knitted fabric (3).

## Revendications

1. Article de contention sans couture qui comprend un tricot de base (3) consistant en au moins un fil à tricoter (4) et au moins un fil de trame (5) élastique qui est inséré ou tricoté dans celui-ci, et présente au moins un logement (1) en forme de tube ou de poche et s'étendant dans une direction longitudinale (L) pour au moins un doigt (F) ou au moins un orteil (Z) d'un porteur de l'article de contention et qui peut être appliqué contre une extrémité de corps du porteur s'étendant dans la direction longitudinale (L), dans lequel le ou chaque logement (1) comprend une pluralité d'élévations (2) en forme de nervure qui se déroulent au moins essentiellement parallèlement les unes aux autres et dans la direction longitudinale (L) du logement (1) respectif, **caractérisé en ce que** les élévations (2) en forme de nervure sont formées par des mailles flottées (F) et/ou des mailles chargées (f) du fil à tricoter (4).

2. Article de contention selon la revendication 1, **caractérisé en ce que** le tricot de base (3) présente un côté intérieur (I) tourné vers l'extrémité de corps quand il est porté et un côté extérieur (A) se situant à l'opposé de celui-ci, dans lequel le tricot de base (3) est tricoté dans une direction de colonne de mailles (s) s'étendant au moins essentiellement dans la direction longitudinale (L) du ou de chaque logement (1) et présente des rangées de mailles (m) se déroulant transversalement à la direction de colonne de mailles (s), dans lequel les élévations (2) en forme de nervure font saillie sur le côté extérieur (A) par-dessus le tricot de base (3).

3. Article de contention selon l'une des revendications précédentes, **caractérisé en ce qu'**entre deux et 30 élévations (2) sont agencées contre chaque logement (1) et/ou **en ce que** les élévations (2) s'étendent dans une rangée de mailles (m) du tricot de base (3) par-dessus au moins deux mailles.

4. Article de contention selon l'une des revendications précédentes, **caractérisé en ce que** des élévations (2) avoisinantes se déroulent parallèlement et à une distance (d) prédéfinie les unes par rapport aux autres, dans lequel au moins deux mailles du fil à tricoter (4) se situent entre deux élévations (2) se succédant dans une rangée de mailles (m).

5. Article de contention selon l'une des revendications précédentes, **caractérisé en ce qu'**un fil de trame (5) se déroule dans chaque rangée de mailles (m) ou dans une rangée de mailles sur deux du tricot de base (3).

6. Article de contention selon l'une des revendications précédentes, **caractérisé en ce que** dans des zones diminuées par pression du tricot de base (3) sont présentes plusieurs rangées de mailles (m) se succédant dans la direction longitudinale (L), dans lesquelles aucun fil de trame ne se déroule.

7. Article de contention selon l'une des revendications précédentes, **caractérisé en ce que** le fil de trame (5) est intégré dans le tricot de base (3) du fil à tricoter (4) en tant que maille chargée (f), entre deux élévations (2) se succédant dans la direction des rangées de mailles (m), dans chaque maille ou dans une maille sur deux.

8. Article de contention selon l'une des revendications précédentes, **caractérisé en ce que** dans une zone de bord des élévations (2) le fil de trame (5) forme deux mailles chargées (f-f) se succédant dans une rangée de mailles (m).

9. Article de contention selon l'une des revendications précédentes, **caractérisé en ce que** dans la direction longitudinale (L) du ou de chaque logement (1) les élévations (2) s'étendent au moins essentiellement sur l'ensemble de l'extension du logement (1) respectif, dans lequel dans les zones de l'article de contention aucune élévation (2) n'est présente à l'extérieur des logements (1).

10. Article de contention selon l'une des revendications précédentes, **caractérisé en ce que** le tricot de base (3) est formé par un tricot avec une couche avant (v) et une couche arrière (h) opposée à la couche avant (v).

11. Article de contention selon la revendication 10, **caractérisé en ce que** la couche avant (v) et la couche arrière (h) contiennent respectivement le fil à tricoter (4), dans lequel le fil à tricoter (4) forme des mailles flottées (F) ou des boucles de charge (f) pour la formation des élévations (2) en forme de nervure, et les emplacements contre lesquels le fils à tricoter (4) se situe en flottant ou forme une boucle de charge (f) sont agencés de façon décalée les uns par rapport aux autres d'au moins une maille, de préférence d'une ou deux mailles, dans des rangées de mailles (m) se succédant respectivement.

12. Article de contention selon l'une des revendications précédentes, **caractérisé en ce que** le fil de trame (5) élastique est un fil guipé avec une âme hautement élastique qui présente une épaisseur dans la plage de 200 à 1500 dtex, ou un fil d'élasthanne ou de caoutchouc.

13. Article de contention selon l'une des revendications précédentes, **caractérisé en ce que** le tricot de base (3) est un tricot droite-gauche et/ou **en ce que** le tricot de base (3) est tricoté dans une armure 1/1.

14. Article de contention selon l'une des revendications précédentes, **caractérisé en ce que** l'article de contention est un gant, dans lequel un logement (1) sert à loger un pouce et/ou au moins un autre logement (1a, 1b, 1c, 1d) sert à loger un doigt, ou **en ce que** l'article de contention est un bas, dans lequel au moins un logement (1) sert à loger un orteil et/ou d'autres logements (1a, 1b, 1c, 1d) servent à loger d'autres orteils.

15. Article de contention selon l'une des revendications précédentes, **caractérisé en ce que** le tricot de base (3) présente un motif de nervure continu sur toute la surface et en complément au moins dans la zone du ou de chaque logement (1) les élévations (2) en forme de nervure, qui font saillie par-dessus le motif de nervure du tricot de base (3).
